# EUROPEAN PATENT APPLICATION

(11) **EP 2 508 172 A1**
(43) Date of publication of application: **10.10.2012**
(21) Application number: 11161396.4
(22) Date of filing: 06.04.2011
(51) Int. Cl.: A61K 9/16, A61K 31/522

(54) **Stable and uniform formulations of entecavir and preparation method thereof**

(71) Applicant: Zentiva, a.s., 102 37 Praha 10 (CZ)
(72) Inventor: Praveen, Khullar, Goa 403 722, Verna (IN); Patel, Shirishbhai, Goa 403 722, Verna (IN); Deolia, Santosh Kumar, Goa 403 722, Verna (IN); Ramesh, Thangaraj, Goa 403 722, Verna (IN); Saravanan, Devarajan, Goa 403 722, Verna (IN); Rao, Dokku Somasundara, Goa 403 722, Verna (IN); Prasad, Kolakaluri Umamaheswara, Goa 403 722, Verna (IN); Keil, Mathias, 102 37, Praha 10 (CZ)
(74) Representative: Sevinç, Erkan

(57) **Abstract**

The present invention relates to a process for producing pharmaceutical formulations for treating Hepatitis B virus infection, comprising entecavir and pharmaceutically acceptable excipients. The process comprises wet granulation of entecavir with pharmaceutically acceptable excipients, and is very suitable to produce low dose solid formulations with improved content uniformity and stability. The invention also pertains to low dose compositions of entecavir with improved content uniformity and stability.

## Description

### Field of the Invention

The present invention relates to pharmaceutical formulations for treating Hepatitis B virus infection, comprising entecavir as pharmaceutically active ingredient and pharmaceutically acceptable excipients. The invention is also pertaining to a new method for producing entecavir formulations suitable for the production of low dosage forms with improved content uniformity and stability.

### Background of the Invention

Entecavir, being a highly potent antiviral agent was disclosed in the U.S. Patent No. 5,206,244 and is currently marketed under the tradename of Baraclude®. Entecavir is also known to be highly potent in treating hepatitis B virus infection, thus very low dosage forms would become sufficient and preferable in treating HBV infections. It was also reported that uses of the compositions containing low dosage entecavir in combination with other pharmaceutically active substances may improve efficacy of the drug. Tablets and capsules are the very common dosage and formulation forms used in the state of the art.

Liquid compositions of entecavir are also disclosed, for instance in WO 2003/086367-A1, wherein entecavir is provided in a pharmaceutically acceptable solvent in an amount ranging from 0.001% to 20% w/v.

High shear granulation process is generally the common way of producing entecavir containing tablets and capsules in the current state of the art. The process, however is too complicated to control quality of products during humidity heat treatment even though ensuring uniform distribution of the active ingredients. Producing the low dose solid entecavir formulations with sufficient homogeneity is of great importance because of the common problems of content uniformity and stability in the course of the process. Therefore, it is currently one of the important challenges of the formulation scientists to provide low dose solid entecavir compositions having improved content uniformity while being sufficiently stable.

EP 1 267 880 A1 discloses a method for producing solid pharmaceutical compositions having entecavir content in the order of 0.001 to 10 mg, comprising dissolving entecavir and an adhesive substance, i.e. povidone, in a solvent to form a solution and then spraying said solution onto a carrier substrate, drying entecavir coated carrier particles, and finally combining said coated particles with desired ingredients to form said pharmaceutical composition. Povidone is mixed with the entecavir solution to improve its adherence uniformity on the carrier substrate. In this process, spraying is also an essential element due to the same objectives. Povidone, also called Polyvinylpyrrolidone (PVP), is a water soluble polymer used as a binder/adhesive agent. However, it at least complicates the process and increases the overall costs of the procedure.

Thus, it is one of the objects of the present invention to provide a new method for producing solid entecavir formulations without using povidone. Another object of the present invention is to provide a method for producing solid entecavir formulations eliminating complicated steps and drawbacks of the prior art. A further object of the present invention is to provide new solid formulations that are useful in treating HBV, and having satisfactory content uniformity and stability even in very low dosages.

These objects have been achieved through the new process for producing the subject formulations as disclosed in claim 1, and a new formulation as disclosed in claim 13.

### Summary of the Invention

According to a first aspect, the invention is directed to a process for producing low dose pharmaceutical formulations for treating Hepatitis B virus infection, comprising entecavir and pharmaceutically acceptable excipients. The process comprises dispersing the entecavir or a pharmaceutically acceptable salt thereof in a granulation liquid comprising purified water to form an entecavir solution, preparing a mixture of excipients, wet granulating of the excipients with the entecavir solution, and then drying the wet blend of the excipients and entecavir solution to form the pharmaceutical composition. Surprisingly, it was found that resulting pharmaceutical formulation showed good results of content uniformity and stability even when binder agents such as povidone of the prior art are not used in the granulation step.

According to the second aspect of the present invention, there is provided new pharmaceutical compositions of entecavir having low dose entecavir content obtainable by the above process. In a preferred embodiment, the composition was prepared by blending of an intra-granular portion comprising entecavir and desired excipients, and an extra-granular portion comprising mainly of the further excipients. It was found that amount of diluents in both portions is critical, and stability of the composition is improved when amount of the diluent in the intra-granular portion is between 1 to 75% by weight of the total composition and in the extra-granular portion an amount between 1 to 15% by weight of the total composition.

### Detailed Description of the Invention

According to the first aspect of the present invention, there is provided a novel process for producing a pharmaceutical composition comprising entecavir or a pharmaceutically acceptable salt thereof with the desired ingredients. According to the second aspect of the present invention, a new composition of entecavir or a pharmaceutically acceptable salt thereof, useful in treatment of HBV is provided. The novel process and composition of the present invention are capable of solving the above objects with minimum overall costs and time.

According to the first aspect as set forth above, the process is very suitable to produce low dose entecavir compositions having entecavir content of 0.001 mg to 10 mg, more preferably from 0.01 mg to 5 mg, and most preferably from 0.5 mg to 1.0 mg. In a preferred embodiment, main objective is to obtain a composition comprising 0.5 mg or 1.0 mg entecavir, or a pharmaceutically acceptable salt thereof pharmacologically equivalent to 0.5 mg or 1.0 mg entecavir base. In another preferred embodiment the pharmaceutically acceptable salt is a monohydrate salt of entecavir.

When carrying out the above objectives, the inventors surprisingly noted that wet granulation of the active substance with pharmaceutically acceptable excipients revealed an improved composition with good performance of content uniformity and product stability which is very important especially for low dose entecavir formulations. The inventors report that content uniformity was further improved by using a rapid mixer granulator (RMG) in preparation of the very low dosage forms.

According to the first aspect of the present invention, the process comprises the steps of dispersing 0.001 mg to 10 mg entecavir or a pharmaceutically acceptable salt thereof in a granulation liquid, preparing a mixture of excipients, and then wet granulating of this mixture directly with the entecavir solution. The granulation liquid as referred above may comprise purified water, a pharmaceutically acceptable solvent such as ethanol and propylene glycole, or a combination thereof. Despite the process presently proposed does not essentially require a binder agent (povidone), starch may successfuly be added to this liquid depending on the types of the excipients and how low is the dosage of the active substance. It was noted that using starch in the granulation liquid may contribute to the content uniformity and stability of the formulation especially in very low dosage forms. The excipients used in this procedure are preferably sifted through a # 30 mesh before being mixed with the entecavir solution.

The mixture of excipients is preferably selected from the group consisting of diluents, disintegrants, lubricants and fillers. In a preferred embodiment the mixture is composed of diluents and disintegrants.

In a further stage of the present process, the entecavir containing particles as obtained with the above procedure are dried, milled and sifted. In a preferred embodiment, dried granules are mixed with an extra-granular portion and lubricated to obtain compressable granules. It was surprisingly found that solid tablets obtained by blending of an intra-granular portion which is prepared by the wet granulation procedure above and an extra-granular portion comprising additional excipients considerably improves stability of the tablets prepared from the resulting mixture when said amount of diluents in the intra-granular portion constitutes 1 to 75% by weight of the total composition whereas in the extra-granular portion constitutes from 1 to 15% by weight of the total composition.

The excipients of the extragranular portion may similarly be selected from the group of diluents, disintegrants, lubricants and fillers.

In the context of the present invention, the term "diluent" refers to any pharmaceutically acceptable material that may be selected from the group of a lactose, lactose monohydrate, cellulose derivatives such as fibrous cellulose, microcrystalline cellulose, calcium phosphate, sugars such as sucrose, dextrose, dextrin, dextrates and glucose, sugar alcohols such as mannitol and sorbitol, with lactose monohydrate, and microcrystalline cellulose being particularly preferred.

Suitable disintegrants as in the context of the present invention include crospovidone, croscarmellose, croscarmellose sodium, sodium starch glycolate, pregelatinized starch, corn starch, low substituted sodiumcarboxymethylcellulose, and mixtures thereof, with crospovidone being particularly preferred.

The lubricant that may be included into the composition is selected from the group consisting of magnesium stearate, stearic acid, sodium stearyl fumarate and sodium lauryl sulfate, with magnesium stearate being preferred.

The resulting composition of the present invention may be compressed into tablets and can be treated with film coating with a coating solution. The solution as such can, for instance be prepared from Opadry® YS-1-7003 and purified water.

To summarize, one of the advantages of the present invention arises in elimination of the binder agents (i.e. povidone) which are essential in the prior art procedures. Another advantage is appearing in improving the content uniformity and stability of the solid compositions especially in very low dosage forms. Still a further advantage of the invention emerges with the novel compositions wherein the composition is formed with intra-granular and extra-granular portions, and amount of diluents in the intra-granular portion constitutes 1-75% by weight of the total composition whereas in the extra-granular portion constitutes 1-15% by weight of the total composition. Further aspects and advantages of the present invention will be apparent to those skilled in the art upon reading the description above along with the following examples which are non-limiting to the scope of the invention.

### Example 1. Preparation of 1 mg entecavir formulation without binding agent

215.31 gm lactose monohydrate, 58.50 gm Avicel ® PH 101 and 25.20 gm crospovidone XL 10 were sifted through a #30 mesh, and were dry mixed in a RMG. 1.59 gm of entecavir monohydrate was separately dispersed in a granulation liquid consisting of 90.00 gm purified water, and resulting granulation solution was poured into the RMG containing the dry-mixed blend of the previously prepared excipients. After completing the granulation, the granulating mass was brought into a fluidized bed drier for obtaining the dried granulates. Then, the dired granules are sifted through a #30 mesh and remnants were milled by a cone mill with 0.5 mm screen. Sifted granules were then mixed with 50.40 gm Avicel ® PH 102 and Crospovidone XL 10 for 10 minutes. The blend was then lubricated with 5.40 gm of magnesium stearate. Subsequently, the blend was compressed by using standard concave punches to form tablets. Tablets were treated with a film coating suspension containing 10.80 gm Opadry® YS-1-7003 and 97.20 gm purified water to obtain the final tablets.

**Table I.**

| **Unit formulation of the tablets prepared according to Example 1** | | |
|---|---|---|
| **Ingredients** | **Function** | **Qty/tab (mg)** |
| **Intragranular portion** | | |
| Entecavir Monohydrate^{#} | API | 1.06 |
| Lactose Monohydrate | Diluent | 143.54 |
| Avicel PH 101 | Diluent | 39.00 |
| Crospovidone XL 10 | Disintegrant | 16.80 |

| **Extra-granular portion** | | |
|---|---|---|
| Avicel PH 102 | Diluent | 33.60 |
| Crospovidone XL 10 | Disintegrant | 2.40 |
| Magnesium Stearate | Lubricant | 3.60 |
| **Weight of the core tablet** | | 240.00 |

| **Film coating** | | |
|---|---|---|
| Opadry YS-1-7003 | Coating agent | 7.20 |
| **Total weight of the tablet** | | 247.20 |
| ^{#}1.06 mg of entecavir monohydrate is eq. to 1 mg of entecavir | | |

### Example 2. Preparation of 1 mg entecavir formulation with starch

Tablets were prepared according to the procedure of Example 1 with the exception that granulation liquid was containing 0.90 gm of starch in 90.00 gm of purified water. Details of the unit formulation obtained with this procedure are given in Table II.

**Table I.**

| **Unit formulation of the tablets prepared according to Example 1 (with starch as viscosity enhancing agent)** | | |
|---|---|---|
| **Ingredients** | **Function** | **Qty/tab (mg)** |
| **Intragranular portion** | | |
| Entecavir Monohydrate^{#} | API | 1.06 |
| Lactose Monohydrate | Diluent | 143.54 |
| Avicel PH 101 | Diluent | 38.40 |
| Crospovidone XL 10 | Disintegrant | 16.80 |
| Starch | Viscosity enh. | 0.60 |

| **Extra-granular portion** | | |
|---|---|---|
| Avicel PH 102 | Diluent | 33.60 |
| Crospovidone XL 10 | Disintegrant | 2.40 |
| Magnesium Stearate | Lubricant | 3.60 |
| **Weight of the core tablet** | | 240.00 |

| **Film coating** | | |
|---|---|---|
| Opadry 03B28796 | Coating agent | 7.20 |
| **Total weight of tablet** | | 247.20 |
| ^{#}1.06 mg of entecavir monohydrate is eq. to 1 mg of entecavir | | |

### Example 3. Stability tests

Tablets of Examples 1 and 2 were subjected to stability study at 55°C for four weeks and impurity content of the samples were observed. Results are given in the following table, wherein the given values represent the amount of impurities as measured with one week intervals.

**Table III.**

| **Stability of the tablets obtained according to Examples 1 and 2** | | | | | | |
|---|---|---|---|---|---|---|
| | RS (%) | Initial | 1 Week | 2 Week | 3 Week | 4 Week |
| **Example 1** | Unknown | 0.072 | 0.073 | 0.079 | 0.072 | 0.070 |
| | Total | 0.16 | 0.155 | 0.173 | 0.176 | 0.211 |

| | RS (%) | Initial | 1 Week | 2 Week | 3 Week | 4 Week |
|---|---|---|---|---|---|---|
| **Example 2** | Unknown | 0.070 | 0.072 | 0.078 | 0.071 | 0.071 |
| | Total | 0.152 | 0.158 | 0.174 | 0.172 | 0.182 |

## Claims

1. A process for producing a solid pharmaceutical composition comprising from 0.001 mg to 10 mg of entecavir or a pharmaceutically acceptable salt thereof, the process comprising the steps of:
a. dispersing the entecavir or pharmaceutically acceptable salt thereof in a granulation liquid consisting of purified water and optionally one or more organic solvents to form an entecavir solution,
b. preparing a mixture of excipients,
c. granulating the excipients with the entecavir solution,
d. drying the wet blend of the excipients and entecavir to form the pharmaceutical composition.

2. A process according to claim 1 wherein the pharmaceutical composition as prepared according to claim 1 constitutes an intra-granular portion, and said process further comprises the step of dry mixing of said pharmaceutical composition with an extra-granular portion consisting of additional excipients.

3. A process according to claim 1 and 2 wherein excipients are selected from the group consisting of diluents, disintegrants, lubricants and fillers.

4. A process according to claim 3 wherein the amount of diluents in the intra-granular portion consitutes 1 to 75% by weight of the total composition whereas in the extra-granular portion constitutes from 1 to 15% by weight of the total composition.

5. A process according to claim 3 wherein diluents are selected from the group consisting of lactose, lactose monohydrate, calcium phosphate, sugars including sucrose, dextrose, dextrin, dextrates and glucose, sugar alcohols including mannitol and sorbitol, cellulose derivatives including fibrous cellulose and microcrystalline cellulose.

6. A process according to claim 3 wherein disintegrants are selected from the group consisting of crospovidone, croscarmellose, croscarmellose sodium, sodium starch glycolate, pregelatinized starch, corn starch, and low substituted sodiumcarboxymethylcellulose.

7. A process according to claim 3 wherein the lubricants are selected from the group consisting of magnesium stearate, stearic acid, sodium stearyl fumarate and sodium lauryl sulfate.

8. A process according to claim 1 or 2 wherein the process further comprises the step of compressing the pharmaceutical composition into tablets.

9. A process according to claim 8 wherein the process further comprises the step of film coating of the tablets with a coating agent.

10. A process according to claim 1 wherein wet granulation is carried out in a rapid mixing granulator.

11. A process according to claim 1 wherein the granulation liquid further comprises starch as a viscosity enhancing agent.

12. A process according to claim 1 wherein the organic solvent of the granulation liquid is ethanol and/or propylene glycole.

13. A process according to claim 1 wherein said pharmaceutically acceptable salt is entecavir monohydrate.

14. A pharmaceutical composition made by mixing of an intra-granular portion formed by a wet granulation and an extra-granular portion, said intra-granular portion comprising from 0.001 mg to 10 mg of entecavir or a pharmaceutically acceptable salt thereof along with a number of excipients, whereas said extra-granular portion being consituted by additional excipients, said excipients in the intra-granular and extra-granular portions being selected from the group consisting of diluents, disintegrants, lubricants and fillers,
wherein the amount of diluents in the intra-granular portion is less than 75% by weight of the total composition and in the extra-granular portion an amount of less than 15% by weight of the total composition.

15. A pharmaceutical composition according to claim 14 wherein said composition comprises 0.5 mg or 1.0 mg of entecavir or equivalent amount of pharmaceutically acceptable salts thereof.

16. A pharmaceutical composition according to claim 14 wherein said pharmaceutical composition is in the form of a tablet having one of the following formulas:
| **Ingredients** | **Qty/tab (mg)** |
|---|---|
| **Intragranular portion** | |
| Entecavir Monohydrate^{#} | 1.06 |
| Lactose Monohydrate | 143.54 |
| Avicel PH 101 | 39.00 |
| Crospovidone XL 10 | 16.80 |
| **Extra-granular portion** | |
|---|---|
| Avicel PH 102 | 33.60 |
| Crospovidone XL 10 | 2.40 |
| Magnesium Stearate | 3.60 |
| **Weight of the core tablet** | 240.00 |
| **Film coating** | |
|---|---|
| Opadry YS-1-7003 | 7.20 |
| **Total weight of the tablet** | 247.20 |
or,
| **Ingredients** | **Qty/tab (mg)** |
|---|---|
| **Intragranular portion** | |
| Entecavir Monohydrate^{#} | 1.06 |
| Lactose Monohydrate | 143.54 |
| Avicel PH 101 | 38.40 |
| Crospovidone XL 10 | 16.80 |
| Starch | 0.60 |
| **Extra-granular portion** | |
|---|---|
| Avicel PH 102 | 33.60 |
| Crospovidone XL 10 | 2.40 |
| Magnesium Stearate | 3.60 |
| **Weight of the core tablet** | 240.00 |
| **Film coating** | |
|---|---|
| Opadry 03B28796 | 7.20 |
| **Total weight of tablet** | 247.20 |
